Europäisches Patentamt

European Patent Office

Office européen des brevets

(19)

(11) Veröffentlichungsnummer: **0 164 629 B1**

# (12) EUROPÄISCHE PATENTSCHRIFT

(45) Veröffentlichungstag der Patentschrift: 24.07.91

(51) Int. Cl.5: **A01N 57/16**

(21) Anmeldenummer: 85106465.9

(22) Anmeldetag: 25.05.85

Die Akte enthält technische Angaben, die nach dem Eingang der Anmeldung eingereicht wurden und die nicht in dieser Patentschrift enthalten sind.

(54) **Verfahren zur Bekämpfung der Varoatose bei Bienen.**

(30) Priorität: 04.06.84 DE 3420751

(43) Veröffentlichungstag der Anmeldung:
18.12.85 Patentblatt 85/51

(45) Bekanntmachung des Hinweises auf die Patenterteilung:
24.07.91 Patentblatt 91/30

(84) Benannte Vertragsstaaten:
**AT BE CH DE FR GB IT LI NL**

(56) Entgegenhaltungen:
**DE-A- 2 208 618**
**GB-C- 2 085 302**

**COMMONWEALTH AGRICULTURAL BUREAU, 1983, Zusammensetzung 83238811, Slough, GB; K.K. HO et al.: "Effects of gubitol and its application methods on honeybee mite (Varroa Jacobsoni Oudemans) in Taiwan" & CHINESE JOURNAL OF ENTOMOLOGY, Band 1, Nr. 1, 1981, Seite 111**

**COMMONWEALTH AGRICULTURAL BUREAU, 1981, Zusammensetzung 81 A31243, Slough, GB; K.K. HO et al.: "Study of chemical control of Varroa JAcobsoni .1. Screen tests of**

miticides on Varroa Jacobsoni and toxicity measurement of five miticides on Apis Mellifera" & NTU PHYTOPATHOLOGIST & ENTOMOLOGIST, Nr. 7, 1980, Seiten 78-83

**APIMONDIA, 1981, Seite 168-179**

**HONEYBEE SCIENCE, 1980, Seite 1-4**

**BIENENSEUCHEN-VERORDNUNG, Artikel 1-4**

(73) Patentinhaber: **BAYER AG**

**W-5090 Leverkusen 1 Bayerwerk(DE)**

(72) Erfinder: **Ritter, Wolfgang. Dr.**
**Welchentalstrasse 14**
**W-7800 Freiburg(DE)**

## Beschreibung

Die vorliegende Erfindung betrifft die Verwendung von 0,0-Diethyl-0-(3-chlor-4-methyl-cumarin-7-yl)-thionophosphorsäureester (Coumaphos) in Form wässriger Suspensionen oder Emulsionen zur Herstellung von Mitteln zur Bekämpfung von Varroa Jacobsoni auf Bienen.

Die Varroatose wird von der Milbe Varroa Jacobsoni hervorgerufen, die ausgewachsen auf Bienen, Drohnen und Bienenköniginnen und in ihren präimaginalen Entwicklungsformen auf Bienen- und Drohnenlarven und den Puppen parasitiert. Zu den von der Milbe direkt hervorgerufenen Schäden tritt eine allgemeine Schwächung der Bienen, die sie anfällig gegen den Befall von Viren und Bakterien macht.

Dieser Parasitismus ist neu in der Bienenzucht und erreicht bereits eine panzootische Form. Durch akute Schwächung und hohe Sterblichkeit der Bienenvölker ruft die Varroatose große Schäden in der Bienenzucht hervor. Auch die unmittelbaren bienenzüchterischen Produkte wie Homig, Wachs, Bienenmilch, Bienengift sind davon betroffen.

Auf Grund der Erniedrigung der Bestäubung der entomophilen landwirtschaftlichen Kulturen beeinträchtigt sie aber auch den mittelbaren Nutzen der Bienen, der 10-15fach die unmittelbare Produktion der Bienen übersteigt.

Bisher sind Methoden zur Bekämpfung der Varroatose bei Bienen bekannt, bei denen die Bienenvölker abgeraucht oder mit chemischen Wirkstoffen bestäubt werden.

Bekannt ist auch die Methode der Thermotherapie und der mechanischen Beseitigung der abgedeckten Drohnenbrut.

Der Nachteil der bekannten Methoden ist der große manuelle Aufwand bei der Anwendung sowie die Beunruhigung der Bienenvölker die von der Behandlung ausgeht.

Aus Honeybee Science 1980, S.155-156 war bekannt 20-30 ml einer wässrigen Lösung von Coumaphos auf jede Wabe mit den darauf sitzenden Bienen zu sprühen. Bei dieser Behandlung kommt es zu durchschnittlich 23,6% Bienenmortalität. Bei Schräghaltung der Waben während des Besprühens kommt es zu einer durchschnittlichen Bienenmortalität von 6,8 %. Die Toxizität gegenüber Bieneneiern und -larven wird mit 29,2 % bzw. 57,5 % angegeben. Das Verfahren ist ohne die Gefahr einer erheblichen Schädigung der Bienen nicht praktisch anwendbar.

In NTU Phytopathologist & Entomologist No. 7,1980, S.78-83 wird ein Prüfverfahren zur Bestimmung der Bienentoxizität von Wirkstoffen beschrieben. U.a. wird Coumaphos geprüft Hinweise zum sicheren Einsatz von Coumaphos in der Praxis werden nicht gegeben.

Aus Vcelastvi 6/83, S.124-125 sind Hinweise über die Verwendung von Coumaphos-haltigen Zuckerlösungen zur Varroatosebekämpfung bekannt. Dabei müssen jedoch verhältnismäßig hohe Wirkstoffmengen eingesetzt werden.

Aus Apimondia 1981, S.168-172 war bekannt, den für diese Anwendung nicht zugelassenen Wirkstoff Chlordimeform Hydrochlorid durch Aufspritzen auf die Wabengassen einzusetzen. Für die praktische Anwendung empfehlen die Autoren jedoch die Fütterungsmethode.

Die vorliegende Erfindung betrifft die Verwendung von 0,0-Diethyl-0-(3-chlor-4-methyl-cumarin-7-yl)-thiono-phosphorsäureester (Coumaphos) in Form einer wäßrigen Suspension oder Emulsion die 100-1000 ppm Wirkstoff enthält zur Herstellung von Mitteln zur Bekämpfung von Varroa Jacobsoni auf Bienen wobei man 10-50 mg Coumaphos pro Behandlung und Bienenvolk durch Aufgießen, Aufträufeln oder Aufspritzen verteilt.

Das Verfahren zur Behandlung wird während der brutarmen Zeit, z.B. während der Überwinterungsphase oder der Ruhephase zwei- bis dreimal im Abstand von 4-20 Tagen bevorzugt im Abstand von 1-2 Wochen durchgeführt.

Für eine Behandlung werden 30-60 ml einer verdünnten wäßrigen Suspension oder Emulsion von Coumaphos pro Bienenvolk verteilt. Bevorzugt sind 50 ml. Die Verteilung erfolgt durch einfaches Aufgießen, Aufträufeln oder Aufspritzen.

Pro Bienenvolk werden dahei 10-50 mg, bevorzugt 15-30 mg Coumaphos ausgebracht.

Die Wirkstoffkonzentration der verdünnten wäßrigen Suspension oder Emulsion beträgt 100-1000 ppm. Bevorzugt beträgt sie 200-700 ppm, besonders bevorzugt ca. 500 ppm.

Zur Herstellung der anwendungsfertigen Lösung wird ein Emulsions oder Supensionskonzentrat mit einer Wirkstoffkonzentration von 0,1-20 %, bevorzugt 10-20 %, besonders bevorzugt 16 % eingesetzt.

Diese Formulierungen werden in bekannter Weise hergestellt, z.B. durch Vermischen des wirkstoffs mit Streckmitteln, also flüssigen Lösungsmitteln, gegebenenfalls unter Verwendung von oberflächenaktiven Mitteln, also Emulgiermitteln und/oder Dispergiermitteln. Im Falle der Benutzung von Wasser als streckmitteln können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline,

2

chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsuloxid, sowie Wasser; als Emulgiermittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es ist nicht erforderlich, durch Zerstäuben, Versprühen oder Verräuchern eine Benetzung jeder Biene sowie ihres gesamten Lebensraums mit der Wirkstofflösung zu erreichen.

Überraschenderweise wirkt Coumaphos sowohl in den Bienen als auch in den Larvenstadien der Bienen systemisch.

D.h. der Wirkstoff wird durch Transport in der Körperflüssigkeit zur an der Biene saugenden Milbe transportiert.

Durch diese Wirkungsweise ist es nicht erforderlich, alle Tiere zu benetzen um so die an ihren saugenden Milben zu treffen. Diese systemische Wirkung bei Bienen war deshalb überraschend, weil Coumaphos bei Warmblütern zur Ektoparasitenbekämpfung angewandt keine systemische Wirkung zeigt.

Im folgenden seien Beispiele für Formulierungen aus denen die anwendungsfertigen Lösungen durch Verdünnen mit Wasser hergestellt werden können angegeben:

## Beispiel 1

Coumaphos                                                           20 g

Emulgator Toximul R (R)   (Mischung aus Alkyl-
                          benzolsulfonat-Ca und
                          nichtionogenen Emulgatoren
                          und Methanol mit Hydro-
                          phil/Lipophil Balance
                          HLB-Wert:    10)                           7 g

Emulgator Toximul S (R)   (Mischung aus Alkylben-
                          zolsulfonat Ca und nicht-
                          ionogenem Emulgator, Me-
                          thanol mit Hydrophil/Li-
                          pophil Balance H L B-Wert:
                          10)                                       .5 g

Solvesso 200 R            (Alkylnaphthalin Gemisch
                          aus hochsiedenden Erdöl-
                          fraktionen)                          ad 100 ml


## Beispiel 2

Coumaphos                                                           16 g

Emulgator 368 (R)         Alkylarylpolyglykol-
                          ether (Molekularge-
                          wicht ca. 1165)                           9 g

Emulgator N P 10 (R)      Nonylphenolpolyglykol-
                          ether                                      9 g

Dimethylformamid                                                   10 g

Solvesso 200                                                   ad 100 ml


## Beispiel 3

Coumaphos                                                            5 g

Emulgator Atlox (R)       (Gemisch aus Polyoxy-
                          ethylenether, Polyoxygly-
                          cerid, Alkylarylsulfonat
                          - sehr leicht wasserlös-
                          lich)                                      4 g

| Emulgator Atlox 3404 ® | (Gemisch aus Polyoxy-ethylenalkylarylether, Alkylarylsulfonat - bildet Emulsion in Wasser) | 2 g |
|---|---|---|
| Emulgator Atlox 3409 ® | (Gemisch aus nichtionischen und anionischen Emulgatoren - löslich in Wasser) | 4 g |
| Solvent PC 2 | (hochsiedende aromatische Erdölfraktion) | ad 100 ml |

## Beispiel 4

| Coumaphos | | 1 g |
|---|---|---|
| Emulgator 368 | | 30 g |
| Dowanol DPM ® | (Dipropylenglykolmethyl-ether) | ad 100 ml |

## Beispiel 5

| Coumaphos | 2,5 g |
|---|---|
| Emulgator 368 | 10 g |
| Emulgator NP 10 | 10 g |
| Solvesso 200 | 20 g |
| Dowanol DPM | ad 100 ml |

## Beispiel 8

| Coumaphos | 2 g |
|---|---|
| Emulgator 368 | 9 g |
| Emulgator NP 10 | 9 g |
| Solvesso 200 | 16 g |
| Dowanol DPM | 45 g |
| Wasser | ad 100 ml |

## Beispiel 7

| | |
|---|---|
| Coumaphos | 1 g |
| Emulgator 368 | 10 g |
| Emulgator NP 10 | 10 g |
| Salvesso 200 | 10 g |
| Dowanol DPM | ad 100 ml |

## Beispiel 8

| | | |
|---|---|---|
| Coumaphos | | 2,5 g |
| Emulgator Tween 80(R) | (Sorbitanmonooleat mit HLB-Wert: 4,3) | 8 g |
| Emulgator Span 80(R) | (Sorbitanmonooleat mit HLB-Wert: 15) | 4 g |
| N-Methylpyrrolidon | | ad 100 ml |

## Beispiel 9

| | | |
|---|---|---|
| Coumaphos | | 1 g |
| Cremophor HS 15(R) | (Polyoxyethylen-600-hydroxystearat) | 20 g |
| Chlorbenzol | | ad 100 ml |

## Beispiel 10

| | | |
|---|---|---|
| Coumaphos | | 1 g |
| Emulgator W(R) | (Alkylarylpolyglykolether, Molekulargewicht ca. 853) | 90 g |
| Dimethylisosorbit | | ad 100 ml |

Die hervorragende Wirkung die mit der erfindungsgemäßen Behandlungsmethode erzielt werden kann, geht aus den folgenden Versuchen hervor:

Beispiel

Bienenvölker der angegebenen Stärke werden durch Aufträufeln von 50 ml einer wäßrigen Emulsion von Coumaphos behandelt. Es werden 5 ml der Emulsion in jede Wabengasse geträufelt. Die Konzentration der Emulsion beträgt dabei 30 ppm, 60 ppm, 90 ppm.

Es werden drei Behandlungen im Abstand von jeweils 5 Tagen durchgeführt. Nach jeder Behandlung wird die Zahl der getöteten Milben und Bienen bestimmt.

Nach der Gesamtbehandlung wurde das Bienenvolk abgetötet. Die Bienen werden gezählt und die noch vorhandenen Milben von den Bienen ausgewaschen.

Es wurde dann die Gesamtzahl der ursprünglich vorhandenen Milben bestimmt. Danach wurde der nach jeder Behandlung erfolgte Grad der Bienen und Milbenmortalität errechnet.

In der folgenden Tabelle sind die Ergebnisse der Versuche im Vergleich mit einem handelsüblichen Mittel angegeben. Das handelsübliche Mittel mit den Handelsnamen Folbex VA® wird dabei vorschriftsmäßig verräuchert. Als Wirkstoff in dem handelsüblichen Mittel ist Isopropyl-4,4'-dibrombenziat enthalten.

Tabelle

| Mittel | Milbenmortalität in % nach Behandlung | | | Bienenmortalität in % nach Behandlung | | |
|---|---|---|---|---|---|---|
| | 1 | 2 | 3 | 1 | 2 | 3 |
| Folbex VA | 40,4 | 67,0 | | 0,1 | 0,1 | |
| erfindungsgemäß | | | | | | |
| Dosis 30 ppm | 78,2 | 93,3 | 98,6 | 0,4 | 0,7 | 1,5 |
| 60 ppm | 91 | 97,5 | 99 | 0,5 | 1,0 | 2,2 |
| 90 ppm | 90,7 | 97 | 100 | 0,5 | 1,0 | 1,0 |

**Patentansprüche**

7

1. Verwendung von 0,0-Diethyl-0-(3-chlor-4-methyl-cumarin-7-yl)-thiono-phosphorsäureester (Coumaphos) in Form einer wässrigen Suspension oder Emulsion, die 100-1000 ppm Wirkstoff enthält zur Herstellung von Mitteln zur Bekämpfung von Varroa Jacobsoni auf Bienen, wobei man 10-50 mg Coumphos pro Behandlung und Bienenvolk durch Aufgießen, Aufträufeln oder Aufspritzen in die Wabengassen verteilt.

## Claims

1. Use of 0,0-diethyl 0-(3-chloro-4-methyl-7-coumarinyl) thionophosphate (coumaphos) in the form of an aqueous suspension or emulsion, which contains 100-1,000 ppm of active compound, for preparing agents for combating Varroa jacobsoni on bees, where 10-50 mg of coumaphos per treatment and bee colony are distributed in the comb ways by pouring on, dropwise application to or spraying on.

## Revendications

1. Utilisation du diéthyl-0,0-(chloro-3-méthyl-4-coumarinyl-7)-0-thionophosphate (Coumaphos) sous forme d'une suspension ou émulsion aqueuse qui contient 100 à 1 000 ppm de substance active pour la fabrication d'agents pour combattre Varroa Jacobsoni chez les abeilles, dans laquelle on répartit 10 à 50 mg de Coumaphos par traitement et par ruche en versant, versant goutte à goutte ou pulvérisant.